# EUROPEAN PATENT APPLICATION

(11) **EP 1 215 614 A1**
(43) Date of publication of application: **19.06.2002**
(21) Application number: 00951872.1
(22) Date of filing: 03.08.2000
(51) Int. Cl.: G06F 19/00, C12Q 1/68, C12N 15/00, G01N 33/50

(54) **METHOD OF RECORDING GENE ANALYSIS DATA**

(30) Priority: 05.08.1999 JP 22250199
(71) Applicant: Takeda Chemical Industries, Ltd., Osaka-shi, Osaka 541-0045 (JP)
(72) Inventor: FUJINO, Masahiko, Takarazuka-shi, Hyogo 665-0805 (JP)
(74) Representative: Wright, Robert Gordon McRae
(86) International application number: JP0005196
(87) International publication number: WO0111533

(57) **Abstract**

This invention relates to a method of recording gene analysis results characterized by analyzing the existence of changes in the base sequence of a donor gene versus the base sequence of a gene that serves as the standard, with respect to a specific gene, and recording on a portable recording medium the distinguishing information unique to said gene and results of the analysis, using an optical recording method.

According to this invention, it is possible to systematically and automatically symbolize data on changes (mutation, variation, polymorphism, etc.) in a gene located on chromosomal genome or mitochondria genome, morbidity risks based thereon, drug responsiveness, side effects, etc. It is also possible to accurately and quickly utilize these data while guarding the patient's privacy.

## Description

### TECHNICAL FIELD

The present invention relates to a method of systematically and automatically symbolizing changes (such as mutations, variations, and polymorphism) in genes located on the chromosome genome or mitochondrial genome and information concerning the morbidity risk, drug responsiveness, side effects, etc., based thereon to allow rapid and accurate utilization of this information while guarding the patient's privacy.

### BACKGROUND ART

The human genome is made up of 3 billion base pairs and exhibits considerable diversity depending on the individual. This diversity appears to be the result of the accumulation and inheritance of rare DNA replication errors that occur during chromosome replication in germ cells. This diversity is classified as changes (mutations) caused by base substitution, insertion or deletion and as changes that occur in only a single base, that occur over a number of bases, and that span a certain length, etc. It is called polymorphism when there are changes in the base sequence of genes ((mutations), sometimes simply referred to hereinafter as gene changes) in at least 1% of the population (at least 10% according to some researchers). It is more often called variation when these changes occur in less than 1%. However, the distinction is not clear. Different combinations of individual or multiple changes in genes express themselves as some type of individual difference or difference in physiological function as a difference in constitution. For example, this is believed to be closely related to differences in the risk of developing a certain disease, differences in responsiveness to various therapeutic techniques or drugs, and differences in side effects caused by drugs.

These changes in genes (mutations, variations, and polymorphism) are known to be intimately related to the development or risk of development of diseases such as diabetes, obesity, hypertension, arteriosclerosis, cancer, hyperlipemia, heart disease, immune-mediated allergy, asthma, bone disease, nerve disease, and Alzheimer's disease. When a change in a single gene is directly associated with the development of a disease such as genetic disease, it is called a disease-causing gene. When changes in individual genes associated with multiple genetic factors increase the risk of contracting a disease, it is called a disease susceptibility gene. Examples are given below.

As concerns gene changes and crisis associated with diabetes, polymorphism of the β3 adrenalin receptors (the no. 64 Trp residue changes to Arg, abbreviated hereinafter as Trp⁶⁴Arg) is frequently encountered in Pima Indians and is known to be a risk factor for obesity (diabetes) (N. Engl. J. Med., 333:343, 1995). A³²⁴³G mutation in the tRNALeu(UUR) gene of the mitochondrial genome is also reported to be related to diabetes (Nat. Genet., 1:368, 1992).

The following examples are known regarding the relationship between gene changes and crisis in hypertension. Mutation by single base substitution (Met²³⁵Thr) on the angiotensinogen gene is demonstrated to have a significant correlation with hypertension (Cell, 71:169, 1992). Single base polymorphism (A¹¹⁶⁵C) of the 3' nontranslation region of the angiotensin II type 1 receptor gene is also reported to correlate with hypertension (Hypertension, 24:63, 1994).

As an example of nerve disease and genetic mutation, polymorphism of the apolipoprotein E gene (ApoE4, Cys¹¹²Arg) is reported as a genetic risk factor for Alzheimer's disease (Science, 261:921, 1993). Elongation of CAG triplet repeats is also known to be a cause of nerve diseases such as Huntington's chorea (Cell, 72:971, 1993).

As examples of arteriorsclerotic disease and changes in genes, mutation of the lipoprotein lipase (LPL) promoter (Proc. Natl. Acad. Scie., USA, 1995, 92:4462) and polymorphism of the apolipoprotein E gene (ApoE2, Arg¹⁵⁸Cys) are reported to be associated with hyperlipemia.

As for cancer, familial breast cancer that is said to account for 5-10% of all breast cancers has been clarified to be due to changes in two disease-causing genes called BRCA1 (Nat. Genet., 8:387, 1994) and BRCA2 (Nature, 378:789, 1995). The changes in these genes are known to be base substitutions within the protein-coding region and nonsense mutations by deletion and frameshift mutations. Changes in the genome that occur in the cells other than the germ cell are called somatic cell mutations. Although they are not passed along to the progeny, somatic cell mutations are well known to cause disorders such as malignant tumors. Mutation of the cancer suppressor gene p53 is reported in osteosarcoma (Lys¹²⁰Ter, Arg²⁸²Trp, Gly²⁴⁵Ser: New Eng. J. Med., 326:1301, 1992) and breast cancer (Arg¹⁸¹His, Cancer Res., 52:3234, 1993).

As for joint diseases, HLA-DRB1⁰0401, which is the allele of major histocompatibility complex (MHC), is reported to be associated with the development of chronic rheumatoid arthritis (J. Clin. Invest., 89:2033, 1992). Osteoarthritis is also reported to be associated with allele 27, which is one of VNTR polymorphism of aggrecan (Osteoarthritis Cartilage, 6:245, 1998).

Asthma is known to develop, for example, due to mutation (Ile⁵⁰Val) of the α-chain of the IL-4 receptors (Nat. Genet., 19:119, 1998) and mutation (Ile¹⁸Leu) of the β-chain of Fc ε RI (Clin. Genetics, 46: 228, 1994).

Type I diabetes is a disease that presents hypoglycemia due to abnormal metabolism of glycogen into glucose. Glucose-6-phosphatase (G6Pase) is identified as the causative gene. Trp¹¹⁸Arg that changes the protein and G²⁷³T mutation associated with splicing aberration of the mRNA are also reported (Am. J. Hum. Genet., 57:549, 1995; Biochem. Biophys. Res. Commun., 248:426, 11998 [sic]).

Changes in genes are also clarified to be intimately related to differences in responsiveness to therapeutic drugs in various diseases and differences in side effects due to certain drugs. For example, the poor clearance of drugs such as isoniazide, phenelzine, and procainamide in polymorphism of N-acetyltransferase found in 40-60% of Caucasians is known to lead to side effects (Science, 281:1820, 1998; Annu. Rev. Pharmacol. Toxicol., 37:268, 1997). A low drug metabolism phenotype encountered in 5-10% of Caucasians is also known to occur in CYP2D6, a drug metabolizing enzyme that belongs to the cytochrome P450 superfamily (Science 281:1820, 1998; Annu. Rev. Pharmacol. Toxicol., 37:268, 1997). Polymorphism of the protein coding region or promoter region of the 5-HT2As serotonin receptor gene is known to be related to the efficacy of clozapine, the antipsychotic (Neurosci. Lett. 217:177, 1996). Changes in the mitochondrial genome are also known to be closely related to diseases and side effects of drugs. For example, the auditory disorders that occur as a side effect of aminoglycoside-based antibiotics such as streptomycin are known to be found in family lines with changes in the mitochondrial genome (A¹⁵⁵⁵G of 12S RNA) (Nat. Genet., 4:289, 1993).

Up to this point, the causative genes of genetic diseases and the like have been identified using polymorphism markers as linkage analysis markers. Specifically, polymorphism markers such as restriction fragment length polymorphism (abbreviated as RFLP), minisatellite polymorphism (also called variable number of tandem repeat (VNTR)), and microsatellite polymorphism have been used to identify the locations of human chromosomal genes (mapping). Restriction fragment length polymorphism is polymorphism that depends on the fragment lengths created by restriction enzyme treatment. It was the first polymorphism employed in linkage analysis. Most forms have two alleles due to substitution of one base and were analyzed primarily by southern hybridization. Minisatellite polymorphism has multiple alleles with different individual alleles in a number of repeating sequences of 7 to 40 bases that make up one unit located in from several hundred to several thousand copies in the genome. In the same way as RFLP, it is generally analyzed by southern hybridization or according to Taq Man method by PCR. Microsatellite polymorphism is polymorphism that appears on the genome as repetition of a sequence of 1 to 4 base pairs and 50-100 thousand are present on the human genome. Since multiple alleles are present and can be identified by PCR, this is currently the major trend in linkage analysis markers. Some 6000 types of microsatellite polymorphism due to a CA repeating sequence in particular have been mapped on the human genome and are often used as linkage analysis markers in the identification of the causative genes of genetic diseases. However, contrary to genetic diseases, many lifestyle diseases are believed to be multifactorial disease due to a combination of multiple disease susceptibility genes. The identification of these disease susceptibility genes requires denser linkage analysis markers than microsatellite polymorphism.

The progress of the human genome project intended to determine the entire base sequence of the human genome and the EST (expression sequence tag) project which provides partial base sequence information on cDNA derived from mRNA from human tissues and cells have gathered a vast amount of information on the base sequence of human genes. As a result, attention has turned to SNP (single nucleotide polymorphism) that appears at a single location in an average of 1000 base pairs (300-500 base pairs according to some researchers) on the human genome as a denser, more effective marker of information on chromosome location than microsatellite polymorphism. There are usually only two alleles in SNP, but linkage analysis is possible by analysis of continuous SNP haplotypes. 150,000-300,000 SNP markers have been mapped among the more than 3 million SNP assumed to be present on the entire human genome and there is an active movement toward using them as indicators to discover disease susceptibility genes, etc. This use of such markers is not limited to analysis of populations of persons related by blood such as familial analysis (linkage analysis conducted using DNA of family members in the case of an evident familial disease such as a genetic disease) or the affected sib pair method (conducted using DNA of only the affected brothers and sisters), but also appears to permit identification of genes associated with biological functions (disease genes and disease susceptibility genes or drug responsiveness and side effects, etc.) through association study that compares other individuals. When SNP is used, qualitative and quantitative changes in the gene where the SNP used as the marker is found are expected to be associated directly with biological function. Even if that is not exactly the case, they will be closely related to genes closely associated with biological function. The target gene can be identified rapidly even in this case given the greater density on the genome than microsatellite polymorphism. This should allow the speedy accumulation of information on disease genes, disease susceptibility genes, and genes associated with drug responsiveness and side effects.

There also exist methods of detecting gene changes (gene test) such as base sequence determination, SSCP (single strand conformation polymorphism), and hybridization using DNA chips. All of these methods permit test using DNA collected from the blood cells, etc. of the subject. Base sequence determination detects changes in DNA by determining the base sequence after amplification by a means such as PCR. SSCP can detect mutations by differences in electrophoretic mobility of single-stranded DNA under nondenaturing conditions following PCR. Hybridization detects mutations by utilizing the fact that hybridization efficiency differs depending on changes in genes. Advances being made in the test techniques are making mass processing relatively easy.

Thus, the findings obtained by studying the changes in genes can be very important in medical treatment in addition to providing an understanding of the characteristics of each individual. Diseases such as lifestyle diseases are multifactorial conditions. As many as 10 to 20 disease susceptibility genes may participate, and there may be multiple changes (mutations, variations, polymorphism) in each of the susceptibility genes. Test of 100 to several hundred genes may become an everyday affair in the near future (Mebio, June issue, p. 79, 1999).

Bar codes are widely used in merchandise and sales control. A bar code is defined by the Automatic Identification Manufacturers as "an automatic identification technique that codes information in a series of parallel, rectangular bars and spaces of varying widths." The American National Standards Institute defines it as "a predetermined pattern of rectangular bars and spaces." There are many types of bar codes, each with its own characteristics. Symbols that suit the intended use and properties of the product are selected and standardized. Codes can sometimes include Roman letters, kana, and kanji in addition to numerical information. An ordinary bar code is a one-dimensional code of symbols and characters arranged in a line, e.g., JAN code (JIS-X0501), ITF code (interleave 2 of 5, JIS-X0502), code 39 (JIS-X0503), code 2 of 5, matrix 2 of 5, and code 128. In contrast to this, there is a two-dimensional bar code in which the symbols and characters or information units corresponding thereto are arranged lengthwise and widthwise. There are two types of two-dimensional bar codes, one which consists of stacked one-dimensional bar codes (e.g., code 49 and PDF417) and a matrix type arranged like pieces arranged on a checkerboard (e.g., DataMatrix, DataCode, QR code, MaxiCode, AztecCode and VeriCode). As a widely used bar code, JAN (Japanese article number) standard type is used primarily in smaller enterprises. In the US medical instruments field, the Health Industry Business Communications Council (HIBCC) used to use code 39 to mark product numbers, manufacturing numbers, expiration dates, etc. However, recently the use of code 128-based UCC/EAN128 has become prevalent. In the Japanese medical industry as well, bar code standardization has been studied and UCC/EAN128 standardized by the HIBCC and EHIBCC (European Health Industry Business Communications Council) has come into use (Recognition of Known Bar Codes and Two-Dimensional Codes, Nippon Kogyo Shuppan, by J. Hiramoto, 1997). Bar codes are also used on ID cards at Kyoto University Hospital (Bar Code Secrecy, by Y. Ozuka, Shokasha, 1996). Thus, the use of bar codes is widespread even in the medical field.

The development of the following has been desired: (1) a classification method that accurately and rapidly characterizes the results of analysis of individual gene changes, (2) an accurate, rapid method of recording and storing changes in genes to estimate the probability of a patient contracting a disease and estimate the optimum mode of treatment at medical treatment facilities such as hospitals, e.g., to predict drug responsiveness and side effects, (3) a means of guarding the privacy of the patient while implementing these methods, and (4) a method of making the individual patient's information portable.

### DISCLOSURE OF THE INVENTION

As a result of diligent research conducted to resolve the aforementioned issues, the present inventors discovered that rapid, appropriate diagnosis and treatment at medical facilities such as hospitals can be made possible by optically recording polymorphism information such as human SNP associated with the patient's risk of contracting a disease, therapeutic drug responsiveness and side effects.

Specifically, the present invention relates to:
(1) A method of recording gene analysis results characterized by analyzing the existence of changes in the base sequence of a donor gene versus the base sequence of a gene that serves as the standard, with respect to a specific gene, and recording on a portable recording medium the distinguishing information unique to said gene and results of the analysis, using an optical recording method,
(2) The method of (1) above characterized by recording on a portable recording medium the unique distinguishing information of the donor of said gene using an optical, magnetic and/or electronic recording method,
(3) The method of (1) above characterized by optically, magnetically and/or electronically recording on a portable recording medium data associated with changes in biological functions that can arise due to changes in the base sequence of said gene,
(4) The method of (1), (2), or (3) above wherein the optical recording method is recording by bar code,
(5) The method of (1) above wherein the change in the base sequence of the gene is a mutation,
(6) The method of (1) above wherein the change in the base sequence of the gene is polymorphism or variation,
(7) The method of (1) above wherein the change in the base sequence of the gene is single nucleotide polymorphism,
(8) The method of (1) above wherein the change in the base sequence of the gene is a change that characterizes that individual,
(9) The method of (1) above wherein the change in the base sequence of the gene is a change that causes a specific disease or permits classification of morbidity risk,
(10) The method of (1) above wherein the change in the base sequence of the gene is a change that permits classification of efficacy of therapeutic drugs,
(11) The method of (1) above wherein the change in the base sequence of the gene is a change that permits classification of the risk of appearance of side effects by specific drugs,
(12) The method of (1) above wherein said gene derives from the chromosome genome,
(13) The method of (1) above wherein said gene derives from the mitochondrial genome,
(14) The method of (1) above wherein said gene derives from a mammal,
(15) The method of (1) above wherein said gene derives from a human.
(16) A system for recording gene analysis data associated with a specific gene, comprising input means to input distinguishing information unique to the gene and the results of gene analysis, data control means to control the input data, and output means to record the input data on a portable recording medium using an optical recording method,
(17) A portable medium, on which distinguishing information unique to a specific gene and the results of analysis have been recorded using an optical recording method by analyzing the existence of changes in the base sequence of a donor gene versus the base sequence of a gene that serves as the standard with respect to said specific gene.

### BRIEF DESCRIPTION OF THE DRAWINGS

Figure 1 is a block diagram that shows the structure of the gene test system.
Figure 2 is a block diagram that shows the test results display system.
Figure 3 is a flow chart that shows data input.
Figure 4 is a flow chart that shows readout of the sample sheet, creation of the gene test protocol, output, and storage.
Figure 5 is a flow chart that shows the suitability of the test data and evaluation of the genotype.
Figure 6 is a flow chart that shows the readout of the test results and display of the test results.
Figure 7 is a flow chart that shows the test protocol (test unit (PLACE-SSCP)).
Figure 8 is an explanatory diagram of an example of the screen structure (screen 1: image).
Figure 9 is an explanatory diagram of an example of the screen structure (screen 2: image).
Figure 10 is an image diagram that shows an example of the test protocol.

1 is the control unit, 2 is the test registration unit, 3 is the test control unit, 4 is the test output unit, 5 is the memory unit, 6 is the display unit, 7 is the data input unit, 8 is the printer unit, 9 is the test unit, 10 is the registration unit, 11 is the search unit, 12 is the test control unit, 13 is the calibration unit, 14 is the result search unit, 15 is the result presentation unit, 16 is the printer unit, 21 is the control unit, 22 is the test results control unit, 23 is the memory unit, 24 is the communications control unit, 25 is the display unit, 26 is the data input unit, 27 is the test results detection unit, 28 is the printer unit, 29 is the web browser, 30 is the evaluation unit, 31 is the printer unit, 41 is the control unit, 42 is the communications control unit, 43 is the data control unit, 44 is the registration unit, 45 is the search unit, 46 is the database, 47 is the web server, and 100 is the network.

### BEST MODE FOR CARRYING OUT OF THE INVENTION

The phrase "gene changes" includes those that occur in only one base on the chromosome genome or mitochondrial genome, those that span multiple bases, and those changes that span a relative length, and changes include deletions, insertions, substitutions, and translocations. These gene changes may be gene variants or gene polymorphism. Examples of gene polymorphism here include RFLP, VNTR polymorphism, microsatellite polymorphism, and SNP. These gene changes may be due to inheritance from the mother or father, may be a maternal inheritance, or may be a somatic cell mutation. These gene changes are preferably associated with information needed by medical facilities. In particular, gene changes associated with a disease, drug responsiveness, or side effects by a drug are preferred. These gene changes are preferably 1) changes in a structural gene corresponding to a gene product (protein or RNA) when differences arise from normal gene products such as bindability with biological components such as RNA, protein, or peptide, intracellular localization of gene products, catalytic activity of gene products, stability of gene products, or affinity for drugs, 2) changes in a region that participates in gene expression such as a promoter/enhancer when a difference arises from normal gene expression, 3) changes in a translation area, etc. when the translation efficiency or mRNA stability differs from that of a normal gene, or 4) changes in an intron, etc. when a difference arises in splicing such that the gene changes directly affect the quality or quantity of gene products and as a result are expressed as differences in biological function as individual differences (such as disease risk, drug responsiveness, or drug side effects). However, even when the gene changes are not directly associated with biological function, they may be changes that can be clearly linked closely to changes in genes that affect biological function through linkage analysis.

Examples of the base sequence of the gene that serves as the standard used in analysis of changes in the donor gene include a normal base sequence or abnormal base sequence. The donor base sequence can be judged to be mutated if there is a difference between a normal base sequence and the base sequence of the donor gene or if there is no difference between an abnormal base sequence and the base sequence of the donor gene.

Examples of the terms "disease" or "condition" include nerve diseases, Alzheimer's disease, schizophrenia, depression, hypertension (e.g., essential hypertension), diabetes (e.g., insulin dependent or nondependent diabetes), obesity, heart disease (e.g., myocardial infarction), cancer (e.g., colon cancer or prostate cancer), bone diseases (e.g., osteoporosis), joint diseases (e.g., osteoarthritis or chronic rheumatoid arthritis), allergic diseases (e.g., atropy), asthma, hyperlipemia, and arteriosclerosis.

Examples of the "portable recording medium" include recording media for optical recording such as paper, label, tape, or card, recording media for magnetic recording such as floppy disk or magnetic card, or recording media for electronic recording such as IC card or IC chip.

The "optical recording method" may be any as long as it is a method of recording that can be recognized visually by humans or a method of recording on a recording medium that can be read out by an optical readout instrument (e.g., bar code reader). However, recording as a bar code is preferred.

Examples of a "bar code" include a one-dimensional bar code of rectangular bars and spaces arranged in a predetermined pattern, a stacked two-dimensional bar code of stacked one-dimensional bar codes, or a two-dimensional matrix bar code of cells constructed of squares or dots arranged lengthwise and widthwise. These bar codes are formed from a quiet zone, start code, data, and stop code and the data may have a parity check function. A check digit may also be added as necessary to lower the error rate. The bar code may meet the standards those of a one-dimensional bar code (e.g., JAN code, ITF code, Code 39, Code 2 of 5, Matrix 2 of 5, or Code 128), two-dimensional stacked bar code (e.g., Code49 or PDF417), two-dimensional matrix bar code (e.g., DataMatrix, DataCode, QR code, MaxiCode, AztecCode, or VeriCode), improved standards based thereon, or newly created standards.

The color of the bar code may be either visible or invisible as long as it can be recognized by a bar code reader (manual scan type, CCD scan type, laser scan type, image type, etc.). For example, when it is visible, it may be a color such as black, blue, red, or green. When it is invisible, it can be read by an infrared light scanner even though invisible as long as the bar code is printed using ink that absorbs only infrared light.

The bar code may be printed directly on the portable recording medium or may be printed on a label, etc. When printed on a label, said label can be made portable by affixing it to a portable object such as a driver's license, credit card, rail pass, or portable telephone.

Examples of magnetic recording methods include floppy disk and magnetic card. Recording on a magnetic card is preferred.

Examples of electronic recording methods include recording on an IC card or IC chip. Recording on an IC card is preferred.

Examples of the data recorded optically include the test categories (such as gene name and location of changes) and test results (such as gene changes, i.e., normal or abnormal type). Since humans are diploid organisms, they have one pair (two) of the same chromosomes. Therefore, the test results of gene changes in autosomes have four possibilities consisting of (W/W), (W/m), (m/W), and (m/m), taking W as normal and m as abnormal. The results can be expressed by three combinations since (W/m) and (m/W) are equivalent. Therefore, the results can be classified by assigning the respective numerical values of "0", "1", and "2" to (W/W), (W/m), and (m/m). Sex chromosomes can be handled in the same way as autosomes in the case of females who are XX. Since males are XY and are formed of two different chromosomes, the changes in one chromosome can be expressed. In other words, there are only two combinations, (W) and (m), in the male X chromosome or Y chromosome. These may be assigned the respective numerical value "0" and "2". Gene changes in either autosomes or sex chromosomes can be expressed by the three numerical values "0", "1", or "2".

Given the great diversity of gene changes, there may be cases in which the results cannot be judged to be normal or abnormal. In this case, the test results may be given a numerical value of "3" as another value. The designation of other may also be given when a test is lost and retesting is not possible. Since the results cannot be judged to be normal or abnormal in these "other" situations, it is equivalent to the situation in which the test was not conducted. Therefore, other situations and untested situations may be assigned the numerical value "3". Consequently, each of the gene changes may be given one of four numerical values consisting of "0", "1", "2", or "3". These can also be recorded in binary form. For example, normal homo (W/W), hetero (W/m), and abnormal homo (m/m) may be expressed as "00", "01", and "11" and untested or other as "10". The test items and information concerning biological functions corresponding to the test results (degree of risk of contracting a disease, drug responsiveness, side effects, etc.) may be input to a computer connected directly or indirectly to the optical recording, magnetic recording, or IC card reader (readout scanner in the case of optical recording and magnetic recording). When there are three or more alleles and biological function (degree of risk of contracting a disease, drug responsiveness, side effects, etc.) differs depending on the allele, each allele may be assigned a number and the number of columns decided so as to permit expression of all combinations expressed as the test results. If it is necessary to distinguish other and untested, for example, one might express other as "3" and untested as "4".

Other information concerning testing such as (1) distinguishing information unique to the donor, e.g., subject's name, insurance no., address, date of birth and other such personal information and (2) the data of testing, test facility, test method, etc., may also be recorded optically, magnetically and/or electronically if necessary.

When personal information and information concerning biological function are recorded magnetically and/or electronically on a portable recording medium, it is preferable to affix a bar code label printed with the test items, results, etc. to said recording medium.

The abbreviations for bases and amino acids that appear in this specification and in the figures are based on the abbreviations of the IUPAC-IUB Commission on Biochemical Nomenclature or on common abbreviations in the field. Examples appear below. When amino acids can have optical isomers, the L form is represented unless specifically indicated otherwise.
- DNA:: deoxyribonucleic acid
- cDNA:: complementary deoxyribonucleic acid
- A:: adenine
- T:: thymine
- G:: guanine
- C:: cytosine
- I:: inosine
- R:: adenine (A) or guanine (G)
- Y:: thymine (T) or cytosine (C)
- M:: adenine (A) or cytosine (C)
- K:: guanine (G) or thymine (T)
- S:: guanine (G) or cytosine (C)
- W:: adenine (A) or thymine (T)
- B:: guanine (G), guanine (G) or thymine (T)
- D:: adenine (A), guanine (G) or thymine (T)
- V:: adenine (A), guanine (G) or cytosine (C)
- RNA:: ribonucleic acid
- mRNA:: messenger ribonucleic acid
- dATP:: deoxyadenosine triphosphate
- dTTP:: deoxythymidine triphosphate
- dGTP:: deoxyguanosine triphosphate
- dCTP:: deoxycytidine triphosphate
- ATP:: adenosine triphosphate
- EDTA:: ethylenediamine tetraacetic acid
- SDS:: sodium dodecylsulfate
- Ter:: termination codon
- Gly:: glycine (G)
- Ala:: alanine (A)
- Val:: valine (V)
- Leu:: leucine (L)
- Ile:: isoleucine (I)
- Ser:: serine (S)
- Thr:: threonine (T)
- Cys:: cysteine (C)
- Met:: methionine (M)
- Glu:: glutamic acid (E)
- Asp:: aspartic acid (D)
- Lys:: lysine (K)
- Arg:: arginine (R)
- His:: histidine (H)
- Phe:: phenylalanine (F)
- Tyr:: tyrosine (Y)
- Trp:: tryptophan (W)
- Pro:: proline (P)
- Asn:: asparagine (N)
- Gln:: glutamine (Q)
- pGlu:: pyroglutamic acid

The sequence numbers in the sequence listing in this specification represent the following sequences.

### [SEQ ID NO: 1]

Shows the base sequence of the primer (synthetic) DNA used in Example 2.

### [SEQ ID NO: 2]

Shows the base sequence of the primer (synthetic) DNA used in Example 2.

### [SEQ ID NO: 3]

Shows the base sequence of the primer (synthetic) DNA used in Example 3.

### [SEQ ID NO: 4]

Shows the base sequence of the primer (synthetic) DNA used in Example 3.

### [SEQ ID NO: 5]

Shows the base sequence of the primer (synthetic) DNA used in Example 4.

### [SEQ ID NO: 6]

Shows the base sequence of the primer (synthetic) DNA used in Example 4.

### [SEQ ID NO: 7]

Shows the base sequence of the primer (synthetic) DNA used in Example 5.

### [SEQ ID NO: 8]

Shows the base sequence of the primer (synthetic) DNA used in Example 5.

### [SEQ ID NO: 9]

Shows the base sequence of the primer (synthetic) DNA used in Example 5.

### [SEQ ID NO: 10]

Shows the base sequence of the primer (synthetic) DNA used in Example 5.

### [SEQ ID NO: 11]

Shows the base sequence of the primer (synthetic) DNA used in Example 5.

### [SEQ ID NO: 12]

Shows the base sequence of the primer (synthetic) DNA used in Example 6.

### [SEQ ID NO: 13]

Shows the base sequence of the primer (synthetic) DNA used in Example 6.

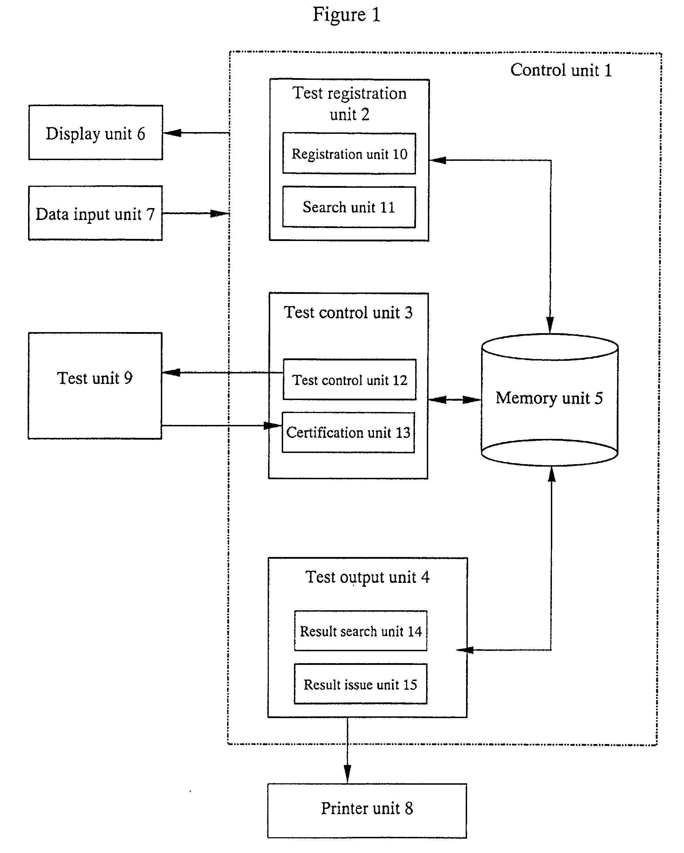

The mode of embodiment of the present invention is described below based on Figures 1-10.

### 1-1. Constitution

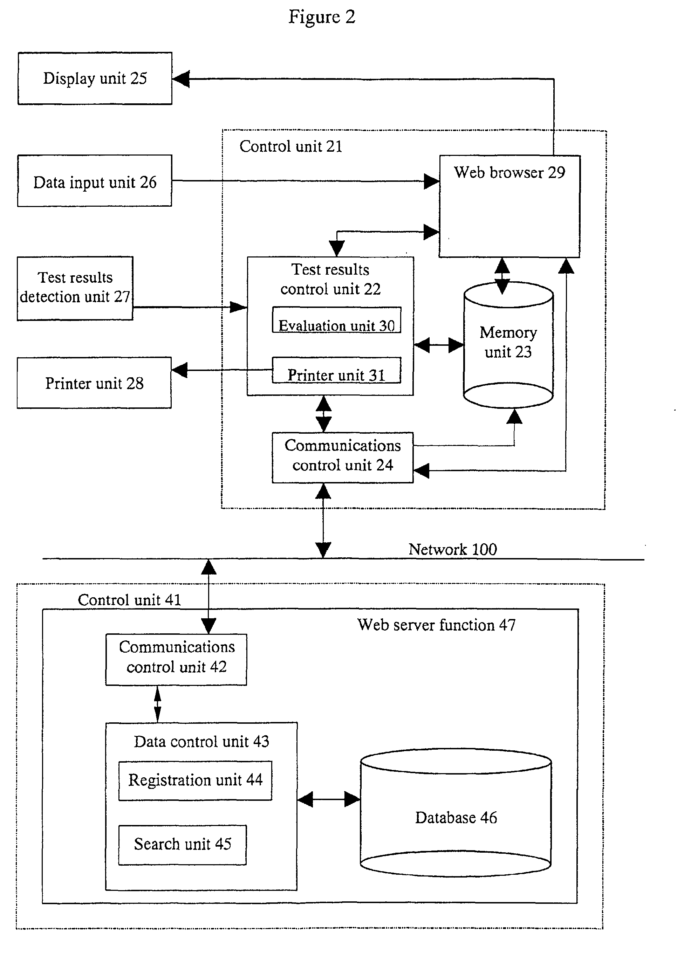

The present invention is constituted from two systems: a gene test system (Figure 1) that employs as the starting materials personal information such as name and date of birth used in ordinary clinical testing, test forms that state the test items, and samples corresponding to the test forms (such as blood) and a test result display system (Figure 2).

### 1-2. Gene test system

This screens the test form that states the personal information such as the name and date of birth and gene test items and sample ID (the sample ID is letters or numbers with no overlap between samples) and the sample stored in a container (refrigerated or frozen as necessary) that states the same ID number as the sample ID number.

The construction of the gene test system will be explained next based on the functional block diagram of Figure 1. For example, control unit 1 consisting of a personal computer and workstation, etc. is equipped with test registration unit 2 to input the subject's personal data, gene test items, and ID number, test control unit 3 to establish the test protocol based on the registered data and verify the test results, test output unit 4 to output the test results, and memory unit 5 to house the data. Control unit 1 is also equipped with display unit 6, data input unit 7, and printer unit 8. Test unit 9 is constituted of the sample processor and test instrument and the personal computer that controls it, a workstation, etc., and is connected by network with control unit 1.

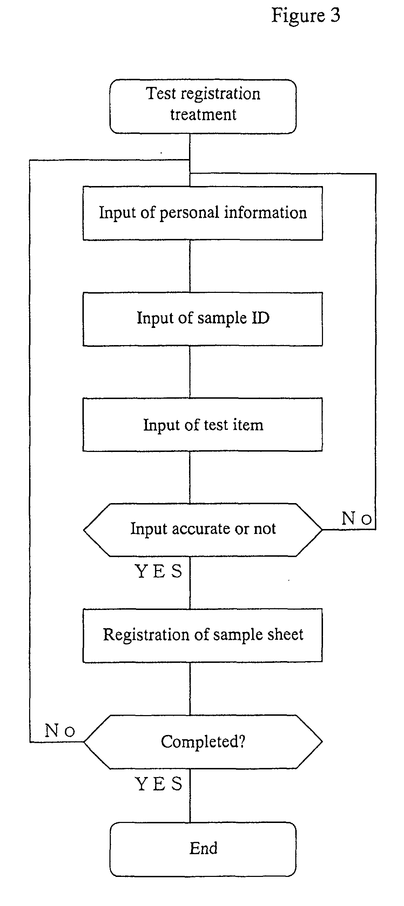

In registration part 10 of test registration unit 2, the data is inputted according to the flow chart shown in Figure 3. Personal information such as the subject's name stated on the test form, the sample ID, and test items are inputted first. Input may be done manually or automatically using a scanner, etc. After confirming the correctness of input, a sample sheet constructed from these data is created and entered in memory. Search unit 11 has the ability to search the entered data.

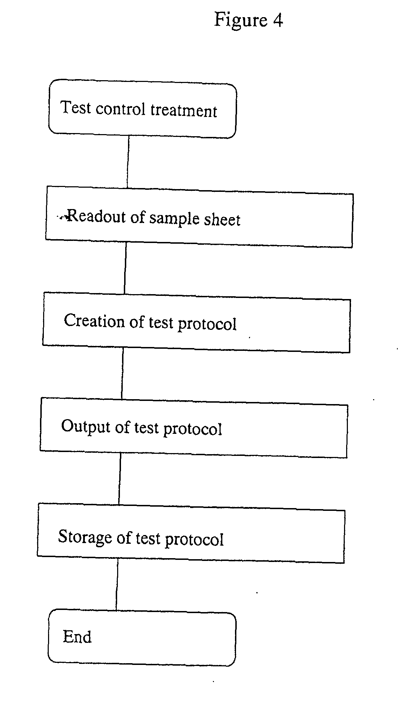

Test control unit 3 creates the test protocol for gene test and evaluates the test results. Test control unit 2 reads the sample sheet input by registration unit 10 according to the flow chart shown in Figure 4, creates a gene test protocol based thereon and outputs to the printer, display, or test unit 9 over the network. The test protocol varies depending on the test method in the test unit, but the sample ID and test items are output so as to correspond to the test results. For example, when testing is conducted by PLACE-SSCP (Genome Research, 7:1094, 1997), it contains information such as (1) plate ID used in PCR, (2) primer used in PCR, (3) PCR reaction conditions, and (4) correspondence of the sample ID number and each well of the PCR plate. This test protocol is stored in memory 5.

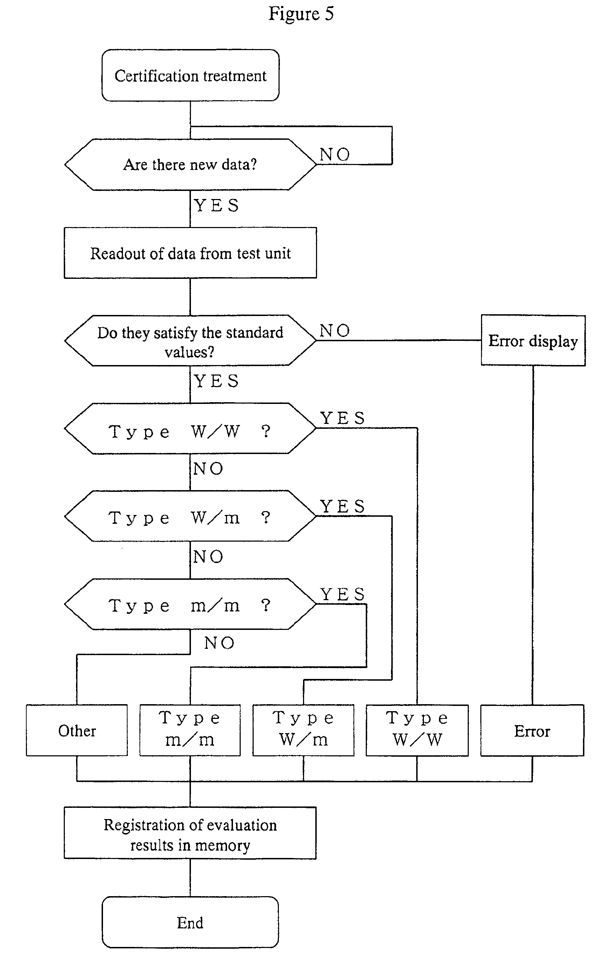

Verification unit 13 reads out the test data from test unit 9 according to the flow chart shown in Figure 5 as long as there are new data and evaluates the appropriateness of the test data and the genotype by the method listed in Figure 5. First it evaluates whether the results meet the standard values. The standard values may be established based on control values in the test or may be established beforehand. Data that match the standard values are evaluated as to genotype (W/W, W/m, m/m, or other) based on the control data in the test, etc. The relevant test protocol is read out from memory 5 and the results of evaluation of genotype entered in memory 5 in correspondence to the sample ID and test items based thereon. When the test does not meet the standard values, an error message is displayed and entered in memory 5 as the result of evaluation.

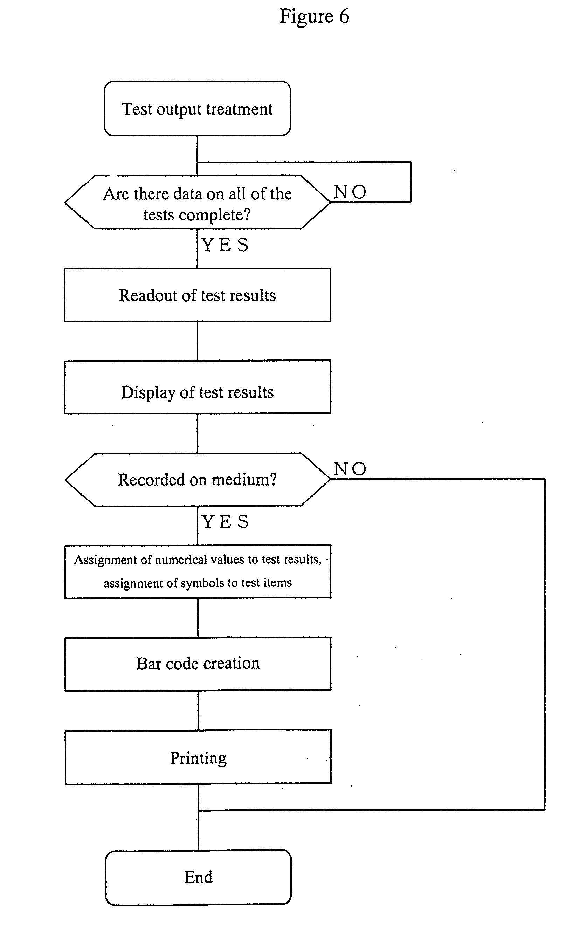

Test output unit 4 reads out the test results from memory 5 by a result evaluation unit as long as there are data that have been completely tested and displays the test results according to the flow chart shown in Figure 6. When recorded (printed) on recording medium, the genotype is assigned numerical values (e.g., W/W = 0, W/m = 1, m/m = 2, other/untested = 3, etc.) in issue unit 15. Based on this, the test results that have been assigned in numerical values are used to create a bar code in accordance with a predetermined procedure using a bar code making and printing software (e.g., Bar Star or Label Start Pro, both made by Ainix) and printed by printer unit 8. Information necessary to identify the donor is also bar coded at this time if necessary. The bar code used here is preferably a two-dimensional bar code with a large information capacity.

Or, one can convert to predetermined test symbols that correspond to and do not overlap the gene test items and state them together with the genotype that has been assigned a numerical value. Test items not performed can be clearly distinguished in this case by not recording the test symbol.

For example, more than 1000 tests can be distinguished by expressing the test symbol by two-character numbers. Since the test results in this case are constructed of a two-character test symbol and a one-character genotype that has been assigned a numerical value, one test can be expressed by three characters. A two-dimensional bar code has a data capacity of more than 1000 characters, making it possible to record more than 300 gene tests in addition to information to identify the donor. Multiple bar codes may be used if it is necessary to record more information than this. Or, the size of the two-dimensional bar code may be increased correspondingly.

Personal information and information on biological function, etc. may be recorded by bar code or may be recorded separately from the test items and test results on a magnetic and/or electronic portable recording medium.

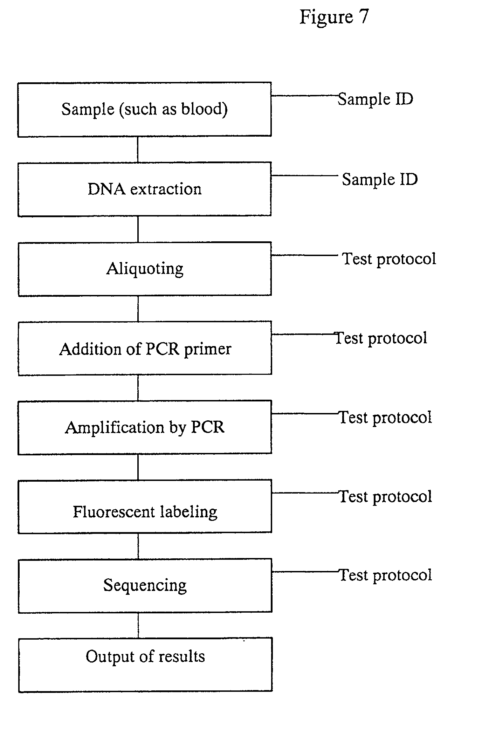

Figure 7 shows a flow chart of the test procedure by PLACE-SSCP (Genome Research, 7:1094, 1997) in the test unit.

Genomic DNA is extracted from the patient's sample (such as blood) by a method such as that stated in Example 1 below. The genomic DNA is given an ID number the same as or corresponding to the sample ID at this time. Next, testing is performed in accordance with the test protocol created by the test control unit (Figure 7). Specifically, the genomic DNA derived from the subject is aliquoted to the designated locations on the designated PCR plate. Next, PCR reaction solution containing the designated primer set is added. A PCR reaction is performed under the designated reaction conditions. After the reaction had been completed, the same plate is submitted to fluorescent labeling and removal of excess fluorescent nucleotide. An internal standard should be added to all samples to correct for fluctuations in electrophoretic mobility. Analysis is performed by GeneScan (PE Applied Biosystems) according to the instructions using a sequencer (ABI Prism 310 Genetic Analyzer, PE Applied Biosystems). The results of analysis by GenScan are output to control unit 1 over the network.

### 1-3. Test results display system

The structure of the test results display system will be explained using the functional block diagram of Figure 2. Control unit 21, constituted of a personal computer and workstation, etc., is consisted of test results control unit 22, memory unit 23, and communications control unit 24. Control unit 21 is equipped with display unit 25, data input unit 26, test results detection unit 27, and printer unit 28. The bar coded gene test results are inputted by test results detection unit 27 composed of an optical reader such as a bar code reader. An error message is displayed when evaluation unit 30 finds an abnormality in input such as damage to the bar code or an accident or erroneous operation of the reader. When input has been performed correctly, evaluation unit 30 displays the gene test results on display unit 25 (e.g., CRT) via web browser 29 based on the test number and information on polymorphism input in memory unit 23. These data can be printed by output to printer unit 28 by printer unit 31. Communications control unit 42 can input various information on polymorphism from database 46 having web server function 47 through network 100.

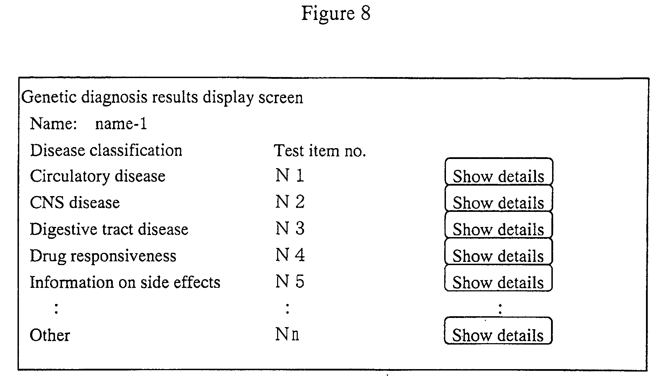

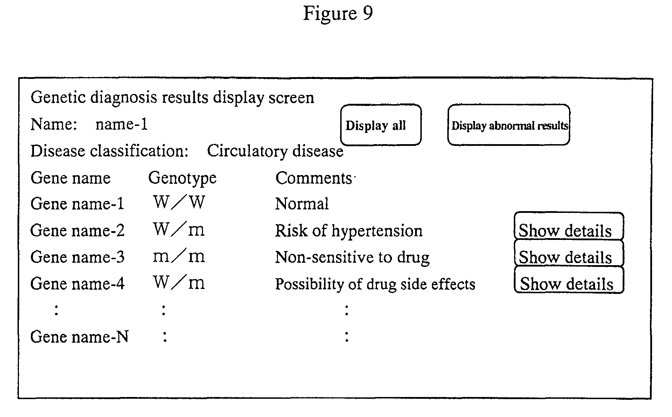

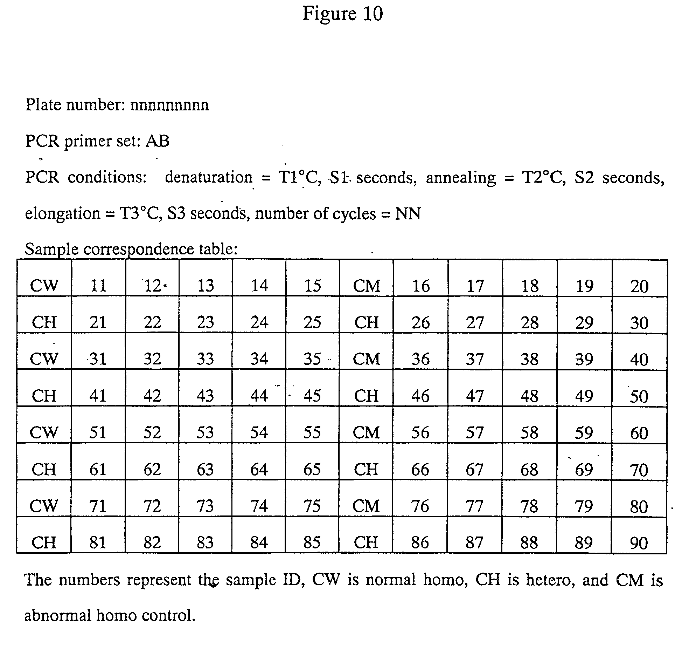

Figures 8 and 9 show examples of the screen constitution of this test results display system. Figure 8 shows a menu screen constitution. The results are classified as disease, drug responsiveness, side effects, etc. on the menu screen and the existence of gene test and test values displayed. By pressing the details button, one obtains a results display screen such as display screen 2 (Figure 9). Figure 10 shows an image diagram of the test protocol.

The present invention is explained in more detail below through Examples. However, these are merely examples and in no way limit the present invention. Furthermore, genetic manipulation using *Escherichia coli* were conducted by the methods described in Molecular Cloning.

### Example 1

### Human DNA specimens

Primarily blood and some arbitrary specimens containing nucleic acid are used. The cells are disrupted and their nucleic acid are released into a medium to stop its degradation.

### Nucleic acid extraction from whole blood

Kits to extract genomic DNA from human whole blood are available from various manufacturers. Here, highly pure DNA is recovered according to the protocol using a DNA extractor WB kit (Nippon Gene). First, 0.5 mL of dissolving solution is added to 0.5 mL of whole blood and mixed. The supernatant is then removed by centrifugation. The sediment is again suspended in 1 mL of dissolving solution and the supernatant is discarded following centrifugation. 200 µL of enzyme reaction solution and 10 µL of protein-degrading enzyme solution are added to the sediment obtained by repeating this procedure one more time. After incubating for 1 hour at 37°C, 300 µL of sodium iodide solution is added. Next, after adding 0.5 mL of isopropanol and mixing, the supernatant is removed by centrifugation. The DNA sediment obtained is washed one time each by wash solutions (A) and (B). It is dissolved in 20 µL of TE solution after drying lightly under reduced pressure.

### Example 2

### Analysis of mutations in the lipoprotein lipase (LPL) gene promoter region

The primers (I and II) shown below are synthesized by the previously reported method [W-S. Yang et al., Proc. Natl. Acad. Scie., USA, Vol. 92, pp. 4462 (1995)] to amplify the LPL promoter gene fragment (563-bp segment of the LPL promoter from +44 to -519) by PCR.

### Sense primer I:

5'-GCTGATCCATCTTGCCAATGTTA-3'
(SEQ ID NO: 1)

### Antisense primer II:

5'-GCAGCTTTCCCTTGAGGAGGAGGAAG-3'
(SEQ ID NO: 2)

The PCR reaction is performed as follows using TaKaRa Ex Taq [Takara Shuzo] and the accompanying buffer. 5 µL of the accompanying PCR buffer (10× concentration), 2 µCi of [α-³²P] dCTP (3000 Ci/mmol); 1 Ci = 37 GBq), 4 µL of dNTP mixture, 0.5 µL (2.5U) of TaKaRa Ex Taq, and the two primers (I and II; 100 pmol each) are added using 2 µL of the genomic DNA solution derived from whole blood obtained in Example 1 as the template. After heating for 2 minutes at 95°C, the PCR reaction is repeated 30 times for 30 seconds at 94°C, 30 seconds at 55°C, and 1 minute at 72°C. The PCR product is separated by 1% agarose gel electrophoresis and the DNA fragment amplified at the location corresponding to the anticipated size (564 bp) is verified.

After further cleaving the amplified DNA fragment by Sau3AI, polymorphism analysis is performed by SSCP primarily according to the method of Orita et al. [Orita et al., Genomics, Vol. 5, p. 874 (1989)]. Following enzymatic digestion, the product is diluted to 1/5-1/10 by 0.1% SDS/10 mM EDTA. After mixing with an equal amount of loading buffer [95% (v/v) formamide/20 mM EDTA/0.05% bromophenol blue/0.05% xylene silanol], it is heated for 3 minutes at 98°C, then quenched on ice. 2 µL of each sample are loaded on 5% native polyacrylamide gel that contains 10% (w/v) glycerol. Each sample is electrophoresed at two gel temperatures (30-31°C and 40-41°C).

Samples with suspected mutations that have different single strand conformers can be discovered by investigating samples from several tens of subjects by the above method. The 564 bp promoter fragment of said samples is amplified by PCR under the conditions described above after removing the radiolabel from its genomic DNA. The band of the amplified segment obtained is then excised after 1% agarose gel electrophoresis and recovered using a QIAquick gel extraction kit (Qiagen). Next, the mutation can be identified by determining the base sequence directly using an ABI Prism Dye Terminator Cycle Sequencing Ready Kit with AmpliTaq DNA polymerase FS (Perkin-Elmer). Mutations such as T⁻³⁹ C and T⁻⁹³ G that occur at the binding site of transcription factor Oct-1 in the promoter region (W-S. Yang et al., Proc. Natl. Acad. Sci., USA, 1995, 92:4462), for example, can be detected by such a search.

### Example 3

### SNP analysis of human p53 gene exon region

SNP of human p53 gene exon 4 can be detected as follows using PLACE-SSCP [Inazuka et al., Genome Research, Vol. 11, p. 1093 (1997)]. PCR primers (III and IV) with the 5'-end sequence modified for fluorescent postlabeling are synthesized using as a reference the previously reported primer sequence for human p53 gene exon [Mashiyama et al., Oncogene, Vol. 6, p. 1313 (1991)].

### Exon 4 sense primer III:

5'-ATTATCTACAGTCCCCCTTGCCG-3'
(SEQ ID NO: 3)

### Exon 4 antisense primer IV:

5'-GTTGCAACTGACCGTGCAAGTCA-3'
(SEQ ID NO: 4)

The PCR reaction is performed as follows using Taq DNA polymerase [PE Applied Systems] and antiTaq antibody [Clontech]. 5 µL of PCR solution containing 50 ng of genomic DNA derived from the aforementioned whole blood as a template, PCR buffer II [PE Applied Systems], 1.5 mM MgCl₂, 0.2 mM 4 dNTPs, 0.5 mM of each of the aforementioned primers, and Taq/AntiTaq (0.125 U) is prepared. After heating for 1 minute at 95°C, a temperature cycle of 30 seconds at 95°C, 30 seconds at 60°C and 1 minute at 72°C is repeated 30 times, followed by heating for 7 minutes at 72°C. Next, a labeling solution containing 0.4 mM each of R110-dUTP and R6G-dCTP [both by PE Applied Systems], 2U of Klenow enzyme [NEB], 20 mM MgCl₂, and 10 mM Tris-HCl is prepared for fluorescence labeling of the PCR product. An equal amount is added to the PCR reaction solution to make 10 µL and reacted for 15 minutes at 37°C. After labeling, the Klenow enzyme is inactivated by adding 1 µL of 0.2M EDTA. 2 U of alkaline phosphatase is added and reacted for 30 minutes at 37°C to degrade the excess fluorescent nucleotide. The fluorescent labeled PCR product is diluted by formamide and denatured by heating for 3 minutes at 95°C. It is then electrophoresed [ABI Prism 310 Genetic Analyzer, PE Applied Systems] by a capillary packed with separatory solution consisting of 5% polymer [PE Applied Systems], 10% glycerol, and 1× TBE buffer. Samples that suggest the presence of SNP based on the electrophoresis wave pattern are again amplified by PCR and the SNP identified by direct sequencing of the PCR product. SNP such as Arg⁷²Pro (CGC CCC) [Ara et al., Nucleic Acids Res., Vol. 18, p. 4961 (1990)], for example, can be detected by such a search.

### Example 4

### SNP analysis in the exon 5 region of the human angiotensin II type 1 (AII type 1) receptor

PCR primers (V and VI) with a T7 or SP6 sequence on the 5' side corresponding to the region of exon 5 are synthesized using as a reference the previously reported base sequence of human angiotensin II 1 (A II type 1) receptor gene [Furuta et al., Biochem. Biophys. Res. Commun., Vol. 183, p. 8 (1992)].

### Sense primer V:

5'-AATTGTAATACGACTCACTATAGGGCCAGCTATAATCCATCGAAA-3'
(SEQ ID NO: 5)

### Antisense primer VI:

5'-AAGCTATTTAGGTGACACTATAGCGAACATGTCACTCAACCTC-3'
(SEQ ID NO: 6)

Using approximately 500 ng of genomic DNA obtained in Example 1 as the template in the PCR reaction, 5 µL of the accompanying PCR buffer (10× concentration), 10 nmol of mixed dNTP, 1 µL of KlenTaq, and two primers (V and VI above; 20 pmol each) are brought to 50 µL by sterilized distilled water. After heating for 5 minutes at 95°C, a cycle of 30 seconds at 95°C, 45 seconds at 60°C, and 45 seconds at 72°C is repeated 35 times, followed by treatment for 7 minutes at 72°C. The excess primer and dNTP in the PCR product obtained are degraded using a PCR product presequencing kit (Amersham). Specifically, 2 µL of exonuclease solution (10 U/µL), and 2 µL of alkaline phosphatase solution (2 U/µL) are added to 10 µL of PCR solution and treated for 15 minutes at 37°C, then for 15 minutes at 80°C. The PCR product thus obtained is reacted directly with commercial T7 and SP6 primers using an ABI Prism Dye Primer Cycle Sequencing Kit. The base sequence is determined using ABI 377. SNP such as T⁵⁷³ C [Bonnardeaux et al., Hypertension, Vol. 24, p. 63 (1994)], for example, can be detected by such a search.

### Example 5

### SNP analysis of N-acetyl transferase 2 (NAT2)

5 PCR primers (VII to XI) [Okumura et al., Clin. Pharmacol. Ther., Vol. 61, p. 509 (1997)] designed based on the previously reported base sequence of human NAT2 gene are synthesized.

### Sense primer VII:

5'-TCAGCCTCAGGTGCCTTGCA-3'
(SEQ ID NO: 7)

### Antisense primer VIII:

5'-AGCATGAATCACTCTGCTTC-3'
(SEQ ID NO: 8)

### Sense primer IX:

5'-CTTAATTCTCATCTCCTGCC-3'
(SEQ ID NO: 9)

### Antisense primer X:

5'-TGTTGGAGACGTCTGCAGGT-3'
(SEQ ID NO: 10)

### Sense primer XI:

5'-ACAATACAGATCTGGTCGAG-3'
(SEQ ID NO: 11)

The PCR reaction is performed using a Gene Amp Reagent Kit (Takara Shuzo). Using approximately 500 ng of genomic DNA obtained in Example 1 as the template, 5 µL of the PCR buffer accompanying the kit (10× concentration), 8 µL of dNTP mixture, 0.5 µL of TaKaRa Ex Taq, and each of two types of primers (the above VII and VIII, IX and X, or XI and XII; 20 pmol each) are added to each tube and brought to 50 µL by sterilized distilled water. After treating for 3 minutes at 94°C, 2 minutes at 60°C, and 3 minutes at 72°C, a cycle of 1 minute at 94°C, 1.5 minute at 60°C, and 1.5 minutes at 72°C is repeated 40 times, followed by treatment for 7 minutes at 72°C.

RFLP is studied as follows. 2 µL of the buffer (10× concentration) appropriate for each restriction enzyme and 0.5 µL of restriction enzyme solution (Kpn I for the PCR product of primers VII and VIII, Taq I for the PCR product of primers IX and X, and BamH I for the PCR product of primers XI and VIII) are added to 2 µL of each PCR product, brought to 20 µL by sterilized distilled water, and incubated for 1 hour at the optimum temperature for each. The digested fragments are analyzed by 1.5% or 2% agarose gel electrophoresis. SNP such as C⁴⁸¹ T (codon 161, silent) if the fragment is not cleaved by Kpn I digestion, SNP such as G⁵⁹⁰ A (Arg¹⁷⁹Gln) with Taq I digestion, and SNP such as G⁸⁵⁷ A (Gly²⁸⁶Glu) by BamH I digestion, for example, can be detected by such a search (Okumura et al., Clin. Pharmacol. Ther., Vol. 61, p. 509 (1997)].

### Example 6

### SNP analysis of apolipoprotein E

The sequence that encodes the region regarded as important in binding of exon 4 to the LDL receptor is amplified by PCR. Two PCR primers (XII to XIII) [Hixson et al., J. Lipid Res., Vol. 31, p. 545 (1990)] designed based on the previously reported base sequence of human apolipoprotein E gene first are synthesized.

### Sense primer XII:

5'-TAAGCTTGGCACGGCTGTCCAAGGA-3'
(SEQ ID NO: 12)

### Antisense primer XIII:

5'-ACAGAATTCGCCCCGGCCTGGTACAC-3'
(SEQ ID NO: 13)

The PCR reaction is performed using a Gene Amp Reagent Kit (Takara Shuzo). Using approximately 250 ng of genomic DNA obtained in Example 1 as the template, 5 µL of the PCR buffer accompanying the kit (10× concentration), 8 µL of dNTP mixture, 0.5 µL of TaKaRa Ex Taq, and the two primers (XII and XIII above; 20 pmol) are brought to 50 µL by sterilized distilled water. After treating for 30 seconds at 97°C, a cycle of 1 minute at 95°C, 1 minute at 60°C, and 2 minutes at 70°C is repeated 35 times, followed by treatment for 7 minutes at 70°C. SNP can be analyzed as follows. After digesting the 244 bp of PCR product obtained by restriction enzyme Cfo I (Boehringer Mannheim), analysis is performed by 8% polyacrylamide gel. Apo E3 (Cys¹¹², Arg¹⁵⁸), apo E2 (Cys¹¹², Cys¹⁵⁸) and apo E4 (Arg¹¹², Arg¹⁵⁸) can be identified by studying the lengths of the bands generated. To investigate other single base mutations, Gly¹²⁷Asp can be detected if the 244 bp of PCR product obtained is digested by the restriction enzyme Taq I and Arg¹³⁶Ser can be detected if it is digested by Hph I [Civeira et al., Atherosclerosis, Vol. 127, p. 273 (1996)].

### Example 7

### Correspondence of test items and test symbols

The polymorphisms and mutations listed in Examples 2-6 correspond to the following test symbols.

**Table 1**

| Test symbol | Gene name (location of polymorphism or mutation) |
|---|---|
| AA | LPL gene promoter (T⁻³⁹C) |
| AB | p53 (Arg⁷²Pro) |
| AC | Angiotensin II type 1 receptor (T⁵⁷³C) |
| AD | N-acetyltransferase 2 (Arg¹⁷⁹Gln) |
| AE | Apolipoprotein E2 (Arg¹⁵⁸Cys) |

### Example 8

### Test results

Changes in the genes of subjects can be investigated using the methods described in Examples 2-6. Since many SNP are biallelic, they can be easily classified as normal and abnormal. The results can be stated as in the following table in correspondence to the test symbols listed in Example 7.

**Table 2.**

| Test results. | | | | | |
|---|---|---|---|---|---|
| Test symbol | AA | AB | AC | AD | |
| Subject 1 | W/W | W/m | m/m | O | W/W |
| Subject 2 | W/m | W/W | W/W | W/m | W/W |
| Subject 3 | m/m | W/W | W/m | W/W | W/W |
| Subject 4 | W/W | m/m | W/W | W/W | W/W |
| Normal is represented as W, abnormal as m, and other as O. | | | | | |

### Example 9

### Bar coding of human gene test (one test result)

As shown in Example 7, the LPL gene promoter of subject 1 was homo normal, subject 2 was hetero, and subject 3 was homo abnormal. As shown in Table 3, for example, the test symbol is shown by two characters composed of English letters and the test result by a single character. Two pairs of normal genes (normal homo) was expressed as 0, one each normal gene and abnormal gene (hetero) as 1, and two pairs of abnormal genes (abnormal homo) as 2. Therefore, subject 1 can be expressed as AA0, subject 2 as AA1, and subject 3 as AA2. These results are bar coded.

**Table 3.**

| Test symbol (two characters) | Test result (one column) |
|---|---|
| 00-XX | 0: normal homo, 1: hetero, 2: abnormal homo, 3: other |

### Example 10

### Standardization of test results and bar coding

The tests are standardized as follows and only the results are bar-coded. Test AA is listed in column 1, AB in column 2, AC in column 3, AD in column 4, and AE in column 5. The test results are expressed in the same way as in Example 9 with normal homo as 0, hetero as 1, abnormal homo as 2, and other as 3. These results can be assigned numerical values as shown in the table. In other words, subject 1 can be expressed as 01230, subject 2 as 10010, subject 3 as 00100, and subject 4 as 02000. These numbers may be bar-coded.

**Table 4.**

| Assignment of numerical values to test results. | | | | | |
|---|---|---|---|---|---|
| | Column 1 (test AA) | Column 2 (test AB) | Column 3 (test AC) | Column 4 (test AD) | Column 5 (test AE) |
| Subject 1 | 0 | 1 | 2 | 3 | 0 |
| Subject 2 | 1 | 0 | 0 | 1 | 0 |
| Subject 3 | 2 | 0 | 1 | 0 | 0 |
| Subject 4 | 0 | 2 | 0 | 0 | 0 |

### Industrial Applicability

The present invention makes it possible to systematically and automatically symbolize changes (such as mutations, variations, and polymorphism) in genes located on the chromosomal genome or mitochondrial genome and information concerning disease risk, drug responsiveness, and side effects, etc., based thereon. It also makes it possible to utilize this information accurately and rapidly while guarding the patient's privacy.

## Claims

1. A method of recording gene analysis results **characterized by** analyzing the existence of changes in the base sequence of a donor gene versus the base sequence of a gene that serves as the standard, with respect to a specific gene, and recording on a portable recording medium the distinguishing information unique to said gene and results of the analysis, using an optical recording method.

2. The method of Claim 1 **characterized by** recording on a portable recording medium the unique distinguishing information of the donor of said gene using an optical, magnetic and/or electronic recording method.

3. The method of Claim 1 **characterized by** optically, magnetically and/or electronically recording on a portable recording medium data associated with changes in biological functions that can arise due to changes in the base sequence of said gene.

4. The method of Claims 1, 2, or 3 wherein the optical recording method is recording by bar code.

5. The method of Claim 1 wherein the change in the base sequence of the gene is a mutation.

6. The method of Claim 1 wherein the change in the base sequence of the gene is polymorphism or variation.

7. The method of Claim 1 wherein the change in the base sequence of the gene is single nucleotide polymorphism.

8. The method of Claim 1 wherein the change in the base sequence of the gene is a change that characterizes that individual.

9. The method of Claim 1 wherein the change in the base sequence of the gene is a change that causes a specific disease or permits classification of morbidity risk.

10. The method of Claim 1 wherein the change in the base sequence of the gene is a change that permits classification of efficacy of therapeutic drugs.

11. The method of Claim 1 wherein the change in the base sequence of the gene is a change that permits classification of the risk of appearance of side effects by specific drugs.

12. The method of Claim 1 wherein said gene derives from the chromosome genome.

13. The method of Claim 1 wherein said gene derives from the mitochondrial genome.

14. The method of Claim 1 wherein said gene derives from a mammal.

15. The method of Claim 1 wherein said gene derives from a human.

16. A system for recording gene analysis data associated with a specific gene, comprising input means to input distinguishing information unique to the gene and the results of gene analysis, data control means to control the input data, and output means to record the input data on a portable recording medium using an optical recording method.

17. A portable medium, on which distinguishing information unique to a specific gene and the results of analysis have been recorded using an optical recording method by analyzing the existence of changes in the base sequence of a donor gene versus the base sequence of a gene that serves as the standard with respect to said specific gene.
